# EUROPÄISCHE PATENTSCHRIFT

(11) **EP 0 385 116 B1**
(45) Veröffentlichungstag und Bekanntmachung des Hinweises auf die Patenterteilung: **07.06.1995**
(21) Anmeldenummer: 90101796.2
(22) Anmeldetag: 30.01.1990
(51) Int. Cl.: A61N 1/375, A61N 1/05

(54) **Herzschrittmacher-Elektrodenzuleitung**
Pacemaker electrode lead
Conducteur d'électrode de stimulateur cardiaque

(30) Priorität: 02.03.1989 DE 3906598
(43) Veröffentlichungstag der Anmeldung: 05.09.1990
(73) Patentinhaber: Osypka, Peter, Dr. Ing., 79639 Grenzach-Wyhlen (DE)
(72) Erfinder: Osypka, Peter, Dr. Ing., 79639 Grenzach-Wyhlen (DE)
(74) Vertreter: Schmitt, Hans, Dipl.-Ing.

(56) Entgegenhaltungen:
- DE-A- 3 304 506
- DE-U- 7 815 518
- DE-U- 8 527 440
- FR-A- 2 465 489
- GB-A- 2 207 375
- US-A- 3 577 622

## Beschreibung

Die Erfindung betrifft eine Herzschrittmacher-Elektrodenzuleitung mit einer Steckvorrichtung, die einen in Längsrichtung hohlen Steckerstift für den Anschluß an einen Herzschrittmacher und eine in Verlängerung des Steckerstiftes eine Einstecköffnung zur Aufnahme des freien Endes der Elektrodenzuleitung aufweisenden Isolierhülle hat, wobei das Elektrodenzuleitungsende durch Verklemmen mit dem Steckerstift elektrisch leitend verbindbar ist.

Es sind bereits verschiedene derartige Steckvorrichtungen bekannt, wobei in der Regel die Verklemmung zwischen Steckerstift und Elektrodenzuleitungsende indirekt dadurch erfolgt, daß der Steckerstift eine Fortsetzungshülse hat, an der in einem Gewinde eine Klemmschraube vorgesehen ist. Dazu ist es erforderlich, daß der Steckerstift bzw. seine Fortsetzung ein entsprechend großes radiales Maß hat, welches bei modernen Herzschrittmachern nicht mehr in deren Einstecköffnung paßt.

Aus der DE-A-33 04 506 ist eine Steckvorrichtung der eingangs erwähnten Art bekannt, bei welcher innerhalb einer Fortsetzungshülse des Steckerstiftes ein als Wendel ausgebildeter Hohlzylinder vorgesehen ist, der seinerseits zum Festklemmen des Elektrodenzuleitungsendes dient, so daß der Steckerstift indirekt oder mittelbar durch Verklemmen dieser Wendel mit dem Elektrodenzuleitungsende verbunden wird. Dabei wird nämlich diese Wendel mit Hilfe einer Klemmschraube verformt wird. Auch dieser Stecker, der in bewährter Weise eine unmittelbare Belastung des wendelförmigen Elektrodenendes vermeidet, hat eine derart große radiale Ausehnung, daß er in die Einstecköffnung moderner Herzschrittmacher nicht paßt.

Moderne Herzschrittmacher haben nämlich aufgrund internationaler Normung und Vereinbarung Einstecköffnungen von erheblich vermindertem Durchmesser, der derzeit etwa 3,2 mm beträgt. Wenn nun ein Herzschrittmacher-Patient einen neuen Herzschrittmacher benötigt, ist es wünschenswert, die schon implantierte Elektrode weiterzuverwenden.

Es besteht deshalb die Aufgabe, eine Steckvorrichtung der eingangs erwähnten Art zu schaffen, die an einer Elektrode und deren Zuleitungsende insbesondere nachträglich z.B. an einer schon implantierten Elektrode, also unter Operationsbedingungen, anbringbar ist und deren Steckerstift in die Aufnahme-oder Einstecköffnung moderner Herzschrittmacher paßt.

Diese Aufgabe wird mit den Mitteln und Merkmalen des kennzeichnenden Teiles des Patentanspruches 1 gelöst.

Dabei macht sich die Erfindung zunutze, daß die Elektrodenzuleitung ohne Isolierung eine Außenabmessung hat, über welche noch ein genügend dünner röhrchenförmiger Steckerstift geschoben werden kann, der dann durch eine bereichsweise radiale Verformung mit diesem Elektrodenende verklemmt werden kann. Für das Anbringen eines solchen Steckers an der Elektrode genügt es also, das Ende dieser Elektrode abzuisolieren, dann die Isolierhülle mit dem Steckerstift über dieses Ende zu schieben, wobei ein von dem Einsteckende in den Steckerstift vorher eingeführtes Stilett als Innenführung hilfreich ist, um dann die Verklemmung nachträglich durchzuführen. Ein überstehender Rest des Elektrodenzuleitungsendes kann an dem Einsteckende des Steckerstiftes abgeschnitten werden.

Da es dabei erforderlich ist, daß dieser neue Stecker mit der Elektrodenzuleitung dicht und vor allem auch isoliert verbunden ist, ist die schon erwähnte Isolierhülle vorgesehen, die an sich aufgrund des üblichen Isoliermateriales, insbesondere Silikon, und aufgrund der ebenfalls möglichst geringen radialen Abmessung eigentlich kaum über die Isolierung des Elektrodenzuleitungsendes geschoben werden kann. Dieses Problem wird durch das zu der Erfindung gehörende Einführelement gelöst, wodurch die Reibung dieser Isolierungen gegeneinander vermindert wird, so daß nach dem Entfernen dieses Einführelementes dann auch die Isolierhülle aufgrund ihres festen Sitzes an der Isolierung des Elektrodenzuleitungsendes zu der stabilen und festen Verbindung beiträgt. Gerade dieses Einführelement, mit welchem die Isolierhülle der Steckvorrichtung vorgefertigt versehen sein kann, erlaubt also eine sehr schnelle und dennoch effektive Verbindung der Steckvorrichtung mit einem Elektrodenzuleitungsende, so daß diese Anbringung eines neuen Steckers an einer schon implantierten Elektrode sehr schnell gleichzeitig mit dem Einsetzen eines neuen Herzschrittmachers erfolgen kann.

Um die Handhabung beim Befestigen dieser Steckvorrichtung zu erleichtern und damit die gesamte Operation zu beschleunigen und dennoch eine feste und dauerhafte Verbindung zu schaffen, ist es zweckmäßig, wenn die Innenlängshöhlung des Steckerstiftes einen über den Außenquerschnitt des abisolierten Elektrodenzuleitungsendes steck- oder schiebbaren Querschnitt hat, wenn der von dem Einsteckende abgewandte rückwärtige Abschnitt des Steckerstiftes von der Isolierhülle umschlossen ist, wenn sich die Isolierhülle über das dem Einsteckende abgewandte Ende des Steckerstiftes fortsetzt und im Inneren dieser Fortsetzung eine Innenhöhlung hat, deren Querschnitt dem Außenquerschnitt der mit Isolierung versehenen Elektrodenzuleitung etwa entspricht,

Das erfindungsgemäß vorgesehene gleitfähige Einführelement erlaubt eine wesentliche Vereinfachung bei der Montage dieser Steckvorrichtung, weil dadurch ein sehr leichtgängiges Einschieben des Elektrodenzuleitungsendes in die Steckvorrichtung und dabei über ein zuvor entgegengesetzt eingeschobenes Stilett möglich ist, ohne daß sich die evtl. zunächst durch chemische oder sonstige Mittel geweitete Isolierhülle gegen ein Einschieben des isolierten Bereiches des Elektrodenzuleitungsendes sträubt oder dieses behindert. Dabei ist wichtig, daß das Einführelement anschließend - aufgrund seiner guten Gleitfähigkeit - leicht herausgezogen werden kann, wodurch dann die inzwischen wieder schrumpfende Isolierhülle in einen festen Reibschluß mit der Isolierung der Elektrodenzuleitung kommt, so daß ganz erhebliche Zugkräfte übertragen werden können, wie sie bei der üblichen Benutzung gar nicht auftreten.

Zum Verformen des Steckerstiftes für seine Verklemmung mit dem in ihm steckenden abisolierten Elektrodenzuleitungsende kann dabei ein Klemmwerkzeug mit einer Öffnung vorgesehen sein, in welche der Steckerstift und sein zu verformender Bereich einführbar ist. Dadurch kann dem Operateur die Fixierung und insbesondere die elektrisch leitende Verbindung zwischen Steckerstift und abisoliertem Elektrodenzuleitungsende erleichtert werden, so daß diese Manipulation nicht mit einer Zange od.dgl. erfolgen muß, bei welcher die Gefahr besteht, daß die Verformung des Steckerstiftes zu weit geht und dieser dann nicht mehr in die Einstecköffnung des Herzschrittmachers paßt.

Das für die Erfindung so wichtige Einführelement besteht zweckmäßigerweise aus zwei trennbaren oder einzelnen Teilen, deren jeder ein aus der Isolierhülle vorstehendes Auszugende hat und in Gebrauchsstellung die Isolierung des Elektrodenzuleitungsendes von der Innenseite der Isolierhülle abschirmt, wobei die Gebrauchsstellung die Position ist, bei welcher dieses Elektrodenzuleitungsende eingeschoben werden soll.

Besonders zweckmäßig ist es dabei, wenn das Einführelement aus einem einstückigen röhrchenförmigen Teil mit in dessen Längsrichtung verlaufender Sollreißstelle gebildet ist, welche durch das Herausziehen gleichzeitig auftrennbar ist. Die Röhrchenform des Einführelementes erlaubt eine bestmögliche Abschirmung der Isoliermaterialien gegeneinander, so daß das Einschieben entsprechend leicht geht. Da jedoch das Röhrchen anschließend von dem Steckerstift weg aus der Isolierhülle herausgezogen werden muß, befindet es sich dann auf der Elektrodenzuleitung, von der es noch entfernt werden muß. Die erwähnte Sollreißstelle bewirkt nun, daß schon beim Herausziehen auch das Auftrennen dieses Röhrchens praktisch von selbst erfolgt, so daß ein nachträgliches Auftrennen und Abnehmen des Röhrchens von der Elektrodenzuleitung eingespart wird.

Wenigstens der röhrchenförmige Teil des Einführelementes kann beispielsweise aus PTFE bestehen. Dieser Werkstoff hat gegenüber Silikon, welches die bevorzugte Isolierung bei Elektrodenzuleitungen und auch bei Isolierhüllen von deren Steckern ist, besonders gute Gleiteigenschaften. Gleichzeitig kann aus diesem Werkstoff ein Röhrchen mit genügend dünner Wandung gefertigt werden, so daß das Einführelement nicht so viel Querschnitt der Innenlängshöhlung der Fortsetzung der Isolierhülle verbraucht, daß allein dadurch schon das Einschieben des isolierten Bereiches des Elektrodenzuleitungsendes behindert würde. Ferner erlaubt die relativ dünne Wandstärke dieses röhrchenförmigen Teiles ein leichtgängiges Aufreißen beim Herausziehen.

An den Auszugenden des Einführelementes können Greifhilfen, insbesondere gegenüber diesen Enden überstehende Verdickungen, Vorsprünge od.dgl. vorgesehen sein, so daß zum Entfernen des Einführelementes keine besonderen Werkzeuge erforderlich sind.

Eine weitere Verbesserung der Steckvorrichtung insbesondere zur Vermeidung von frühzeitigen Brüchen im Steckerbereich kann darin bestehen, daß zwischen dem von dem Herzschrittmacher abgewandten Ende des Steckerstiftes und der zur Aufnahme der Isolierung des Elektrodenzuleitungsendes dienenden Fortsetzung der Isolierhülle ein Zwischenabschnitt der Isolierhülle vorgesehen ist, dessen Innenhöhlung einen Querschnitt hat, der dem Außenquerschnitt der abisolierten Elektrode oder Elektrodenwendel etwa entspricht. Dadurch wird erreicht, daß in Fortsetzung des Steckerstiftes und somit etwas außerhalb des Herzschrittmachers zunächst ein Bereich der Elektrodenzuleitung von der Isolierhülle umschlossen ist, der seinerseits keine Isolierung mehr hat, so daß der montierte Stecker in diesem Bereich eine größere Flexibilität oder Biegsamkeit aufweist. Körperbewegungen und dergleichen können somit wesentlich besser ausgeglichen werden.

Es wurde schon das zu der Steckvorrichtung gehörende und passende Klemmwerkzeug erwähnt. Zweckmäßig ist es dabei, wenn ein mit einer Stecköffnung versehenes Haltestück vorgesehen ist, dessen Stecköffnung auf das Einsteckende des Steckerstiftes paßt und vor dem Einstecken des Steckerstiftes in den Herzschrittmacher von dem Steckerstiftende abziehbar ist, und wenn der Innenquerschnitt der Stecköffnung des Haltestückes die Außenseite des Steckerstiftes in einem Schiebe- oder Klemmsitz umschließt und wenn schließlich an dem Haltestück wenigstens ein radial bis in den Querschnitt der Stecköffnung des Haltestückes verstellbares Klemmelement vorgesehen ist. Wird nun mit Hilfe dieses Klemmelementes die radiale Verformung des Steckerstiftes für dessen elektrisch leitende Verbindung mit dem Elektrodenzuleitungsende durchgeführt, kann sich der Steckerstift nicht in Reaktion dieser Verformung radial nach einer anderen Seite hin nach außen verformen, da er von der Stecköffnung des Haltestückes fest umschlossen ist. Somit wird auf einfache Weise verhindert, daß die Verklemmung zwischen Steckerstift und Elektrodenzuleitungsende den Steckerstift in seinen Außenabmessungen verändert; somit bleibt gewährleistet, daß er auch nach dieser Operation in die Stecköffnung der Herzschrittmachers paßt.

Das radial verstellbare Klemmelement kann mit Hilfe von Gewindegängen relativ zu dem Haltestück verstellbar sein und hat vorzugsweise an einem außerhalb des Haltestückes befindlichen Bereich oder Ende einen Drehgriff. Somt kann der Operateur die radiale Verstellung sehr fein dosieren und benötigt dazu kein weiteres Drehwerkzeug.

Damit die Verformung des Steckerstiftes gezielt und bei gleichzeitiger Drehung des oder der Klemmelemente erfolgen kann, ist es zweckmäßig, wenn das/die Klemmelemente am in den Querschnitt der Stecköffnung des Haltestückes verstellbaren Ende angespitzt ist/sind und die Spitze, vorzugsweise Kegelform hat, so daß das Klemmelement auch beim Eindringen in die Stecköffnung und radialen Verformen des Steckerstiftes drehbar bleibt.

Besonders günstig ist es, wenn zwei einander an dem Haltestück gegenüberliegende und vorzugsweise etwa miteinander fluchtende Klemmelemente oder Klemmschrauben vorgesehen sind. Sie können dann praktisch gleichzeitig mit je einer Hand erfaßt und aufeinanderzubewegt werden. Dadurch ergibt sich auch eine symmetrische Verformung des Steckerstiftes und eine entsprechend feste und elektrisch leitende Verbindung zwischen diesem Steckerstift und dem zuvor eingeschobenen Elektrodenzuleitungsende.

Zwischen der Außenseite des Haltestückes und einem Anschlag, insbesondere dem Drehgriff des Klemmelementes kann eine die radiale Verstellung des Klemmelementes in das Haltestück und dessen Stecköffnung hinein begrenzender Abstandhalter vorgesehen sein. Damit wird sichergestellt, daß die Verformung mit einem vorgegebenen Maß erfolgt, welches nicht überschritten werden kann, so daß die Gefahr einer Zerstörung des Steckerstiftes bei dieser Manipulation praktisch ausgeschlossen ist.

Zur weiteren Beschleunigung der Monatage kann das Haltestück mit dem Steckerstift und dessen Isolierhülle vorgefertigt lösbar insbesondere mit Hilfe des/der Klemmelemente verbunden sein, wobei das/die Klemmelemente den Steckerstift reibschlüssig, unterhalb seiner Verformungsschwelle beaufschlagen. Es werden also die zum Verformen des Steckerstiftes dienenden Klemmelemente zunächst dazu verwendet, das Klemmwerkzeug von vorneherein vorgefertigt mit dem Steckerstift zu kuppeln und zu verbinden, so daß der Operateur sich zunächst ganz darauf konzentrieren kann, das abisolierte Elektrodenzuleitungsende und den noch isolierten Bereich in die Steckervorrichtung einzuschieben, wonach er sofort auch die Verklemmung durchführen kann, ohne nun zunächst noch ein Werkzeug angereicht zu bekommen. Dabei ist günstig, daß das Haltestück den Steckerstift umschließt, so daß der Operateur beim Einschieben des Elektrodenzuleitungsendes dieses Haltestück auch zum indirekten Halten der Steckvorrichtung benutzen kann und nicht an dem sehr dünnen Steckerstift oder gar der Isolierhülle anfassen muß, die dadurch evtl. so zusammengedrückt würde, daß sie dem Einschieben des Elektrodenzuleitungsendes einen zusätzlichen Widerstand entgegensetzen würde. Somit bekommt das Klemmwerkzeug und insbesondere dessen Haltestück eine zusätzliche wichtige Funktion.

An dem Haltestück, insbesondere einem Haltezylinder, kann ein vorzugsweise durchsichtiges Schutzröhrchen angeschlossen sein, welches wenigstens einen Teil der Isolierhülle umschließt und vorzugsweise bis über das Ende der Isolierhülle reicht. Dadurch wird die flexible und somit empfindliche Isolierhülle der Steckvorrichtung bei deren Lagerung und Versand zusätzlich geschützt. Ferner bekommt dadurch das Klemmwerkzeug und sein Haltestück eine weitere wichtige und vorteilhafte Funktion, da es gewissermaßen als Verpackung vor allem der Isolierhülle dienen kann.

Es sei noch erwähnt, daß eine Abstufung am Übergang von dem größeren Innenquerschnitt der Isolierhülle zu dem nächst kleineren als Anschlag für die radial gegenüber der Elektrode überstehende Elektrodenisolierung dient. Dies gibt dem Operateur eine Hilfe dafür, daß das Elektrodenzuleitungsende und der noch isolierte Bereich genügend tief in die Steckvorrichtung und deren Isolierhülle eingeschoben ist.

Insgesamt ergibt sich eine Steckvorrichtung, die aufgrund von mit ihr bevorzugt vorgefertigt verbundenen Zusatzalementen wie dem Einführelement und auch dem Klemmwerkzeug sehr schnell und einfach mit einem Elektrodenzuleitungsende verbunden werden kann, wobei sich eine sehr feste und stabile Verbindung herstellen läßt, die gleichzeitig eine sehr gute Isolierung ergibt, aber in den Außenabmessungen gegenüber herkömmlichen Steckvorrichtungen derart vermindert ist, daß sie zu modernen Herzschrittmachern paßt und auch der außerhalb des Herzschrittmachers verbleibende Teil innerhalb des Körpers weniger Raum einnimmt.

Nachstehend ist ein Ausführungsbeispiel der Erfindung mit ihren wesentlichen Einzelheiten anhand der Zeichnung näher beschrieben.

Es zeigt in zum Teil schematisierter Darstellung:
- Fig. 1: eine erfindungsgemäße Steckervorrichtung mit daran vorläufig befestigtem Klemmwerkzeug und Einführelement, wobei ein Führungsstilett bereits eingeführt ist und ein teilweise abisoliertes Elektrodenzuleitungsende eingeschoben wird,
- Fig. 2: eine der Fig. 1 entsprechende Darstellung nach dem Einschieben des Elektrodenzuleitungsendes beim Verklemmen des Steckerstiftes mit dem absisolierten Bereich des Elektrodenzuleitungsendes, welcher dabei im Inneren von dem noch liegenden Stilett abgestützt ist,
- Fig. 3: in vergrößertem Maßstab die in Fig. 2 durch einen strichpunktierten Kreis markierte Einzelheit der eigentlichen Klemmvorrichtung des Klemmwerkzeuges,
- Fig. 4: eine erfindungsgemäße Steckervorrichtung mit eingeführtem Stilett ohne Klemmwerkzeug,
- Fig. 5: teilweise im Längsschnitt die Klemmvorrichtung mit einem Halteteil, den radial verstellbaren Klemmelementen und an deren Außenseite angeordneten Abstandhaltern und einem an dem Haltestück angeordneten Schutzröhrchen zur Aufnahme der Isolierhülle, wobei in teilweisem Längsschnitt auch der eigentliche Steckerstift der Steckvorrichtung dargestellt ist,
sowie
- Fig. 6: in vergrößertem Maßstab die erfindungsgemäße Steckvorrichtung nach ihrem befestigen an dem Elektrodenzuleitungsende, wobei Stilett und Klemmwerkzeug noch vorhanden sind und das Einführelement mit Hilfe von Greifhilfen gerade aus der Isolierhülle herausgezogen ist.

Eine im ganzen mit 1 bezeichnete Steckvorrichtung dient dazu, eine Herzschrittmacher-Elektrodenzuleitung 2 mit einem in den Zeichnungen nicht dargestellten Herzschrittmacher kuppeln zu können. Dabei soll diese Steckvorrichtung 1 vor allem dazu geeignet sein, nachträglich an einer solchen Elektrodenzuleitung 2 angebracht zu werden, wenn nämlich beispielsweise ein herkömmlicher Herzschrittmacher eines Patienten gegen einen modernen Herzschrittmacher mit wesentlich kleinerer Einstecköffnung ausgetauscht, die schon implantierte Elektrodenzuleitung 2 aber weiter verwendet werden soll, oder wenn an einer Elektrodenzuleitung eine bisherige Steckvorrichtung durch Leitungsbruch od.dgl. ersetzt werden soll. Gegebenenfalls könnte natürlich die Steckvorrichtung 1 auch von vorneherein an einer Elektrodenzuleitung 2 bei deren erstmaliger Benutzung angebracht sein oder werden.

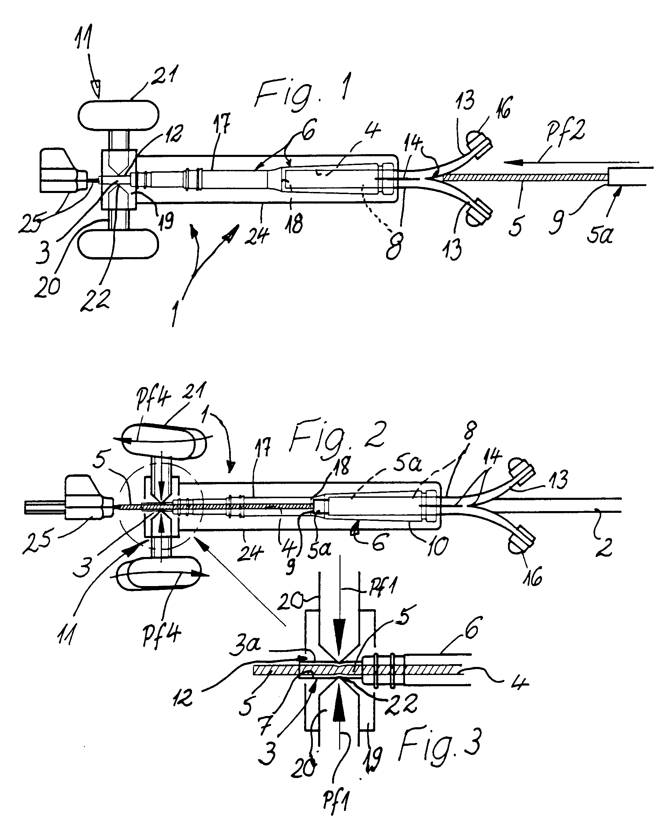

Zu der Steckvorrichtung 1 gehört vor allem ein in Längsrichtung hohler, röhrchenförmiger Steckerstift 3, den man besonders gut in den Figuren 3, 5 und 6 erkennt. Dieser Steckerstift 3 stellt den Anschluß an einen Herzschrittmacher her und soll in dessen Steck- oder Kontaktöffnung passen. In Verlängerung dieses Steckerstiftes 3 ist eine eine Einstecköffnung 4 zur Aufnahme des freien Endes 5 der Elektrodenzuleitung 2 aufweisende Isolierhülle 6 angeordnet, wobei das Elektrodenzuleitungsende 5 in noch zu beschreibender Weise durch Verklemmen mit dem Steckerstift 3 elektrisch leitend verbunden werden kann.

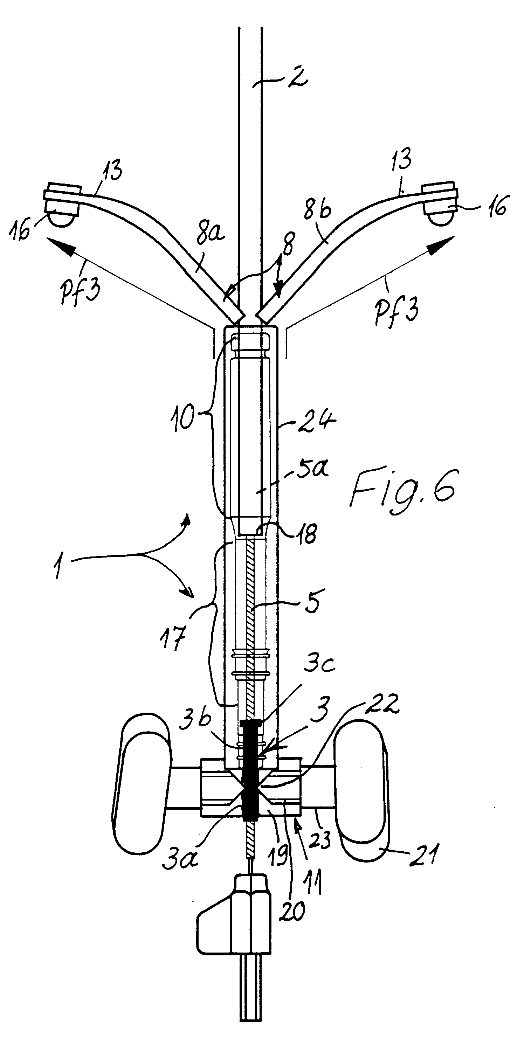

Vor allem in Fig. 3, aber auch in Fig. 2 erkennt man, daß die Innenlängshöhlung 7 (vgl. auch Fig. 5) des hohlen oder röhrchenförmigen Steckerstiftes 3 über das abisolierte Elektrodenzuleitungsende 5 paßt und zum Verklemmen damit bereichsweise gemäß den Pfeilen PF1 radial verformbar ist. Gemäß Fig. 1, 2, 4 und 6 trägt dabei der Steckerstift 3 an seinem dem Einsteckende abgewandten Ende die Isolierhülle 6 mit einem Einführelement 8 für den isolierten Bereich 5a des Elektrodenzuleitungsendes 5. Dadurch soll erreicht werden, daß das Elektrodenzuleitungsende 5 über einen gewissen Restbereich seine Isolierung 9 behalten kann und diese auch von der Isolierhülle 6 umschlossen wird, um im Bereich der Steckvorrichtung 1 eine dichte Isolierung auch bei nachträglicher Anbringung der Steckvorrichtung 1 zu erzielen. Das Einführelement 8 erlaubt nun das Einschieben auch der Isolierung 9 des Bereiches 5a des Elektrodenzuleitungsendes 5 in das Innere der Isolierhülle 6, obwohl normalerweise die isolierenden Werkstoffe insbesondere bei engen Toleranzen nicht ineinander schiebbar oder steckbar wären. Dies gilt vor allem dann, wenn als Isolierwerkstoff der für solche Steckvorrichtungen 1 und Elektrodenzuleitungen 2 bewährte Werkstoff Silikon verwendet wird.

Im einzelnen ist vorgesehen, daß die Innenöffnung 7 des Steckerstiftes 3 einen über den Außenquerschnitt des abisolierten Elektrodenzuleitungsendes 5 steck- oder schiebbaren Querschnitt hat, daß der von dem Einsteckende 3a abgewandte rückwärtige Abschnitt 3b des Steckerstiftes 3, der gegebenenfalls noch eine Querschnittsvergrößerung 3c zur besseren Befestigung haben kann, von der Isolierhülse 6 umschlossen ist und sich diese Isolierhülse 6 über das dem Einsteckende 3a abgewandte Ende 3b des Steckerstiftes 3 fortsetzt. Im Inneren dieser Fortsetzung 10 setzt sich auch die Innenlängshöhlung 4 der Isolierhülle 6 fort, wobei der Querschnitt in dieser Fortsetzung 10 dem Außenquerschnitt der mit der Isolierung 9 versehenen Elektrodenzuleitung 2 etwa entspricht. In diese Fortsetzung 10 ist nun das schon erwähnte Einführelement 8 so eingefügt, daß es diese Innenhöhlung 4 wenigstens teilweise auskleidet. Dabei besteht das Einführelement 8 aus einem Werkstoff, gegenüber welchem die Außenisolierung 9 der Elektrodenzuleitung 2 gleitfähig ist. Das Einführelement 8 steht über das dem Einsteckende 3a abgewandte rückwärtige Ende der Isolierhülle 6 vor und kann nach dem Einschieben des Elektrodenzuleitungsendes 5 relativ zu diesem und zu der Isolierhülle 6 entgegen der durch den Pfeil Pf2 in Fig. 1 angedeuteten Einsteckrichtung herausgezogen werden. Der den abisolierten Bereich des Elektrodenzuleitungsendes 5, also die eigentliche wendelförmige Elektrodenzuleitung, in sich aufnehmende Teil des Steckerstiftes 3 ist zum Verklemmen mit dem Elektrodenzuleitungsende 5 und damit zur elektrisch leitenden Verbindung in noch zu beschreibender Weise radial verformbar.

Zum Zwecke dieser Verformung des Steckerstiftes 3 ist ein im ganzen mit 11 bezeichnetes Klemmwerkzeug mit einer Öffnung 12 vorgesehen, in welche der Steckerstift 3 und sein zu verformender Bereich einführbar ist.

Das für die Anbringung der Steckvorrichtung 1 am Elektrodenzuleitungsende 5 ebenfalls äußerst wichtige Einführelement 8 besteht aus zwei trennbaren oder einzelnen Teilen 8a und 8b, wie es insbesondere in Fig. 6 verdeutlicht ist, deren jeder Teil ein aus der Isolierhülle 6 vorstehendes Auszugende 13 hat und in Gebrauchsstellung die Isolierung 9 des Elektrodenzuleitungsendes 5a von der Innenseite der Isolierhülle 6 abschirmt. Bevorzugt ist dabei dieses Einführelement 8 aus einem einstückigen röhrchenförmigen Teil mit wenigstens einer, bevorzugt zwei in dessen Längsrichtung verlaufenden Sollreißstellen 14 gebildet, welche durch das Herausziehen des Einführelementes 8 gemäß den Pfeilen Pf3 in Fig. 6 gleichzeitig auf- bzw. auseinandertrennbar sind. Nach dem Einführen des Elektrodenzuleitungsendes 5 mit seinem isolierten Bereich 5a in die Isolierhülle 6 und der Verklemmung innerhalb des Steckerstiftes 3 kann also das Einführelement 8 aus der Isolierhülle 6 herausgezogen werden, wodurch allerdings sein röhrchenförmiger Bereich auf die Elektrodenzuleitung 2 gelangt, wo er natürlich nicht bleiben kann. Durch die Sollreißlinien 14 wird aber gleichzeitig auch die Lösung des Einführelementes 8 von der Elektrodenzuleitung 2 bewerkstelligt.

Wenigstens der röhrchenförmige Teil des Einführelementes 8, bevorzugt natürlich das ganze Einführelement 8, besteht dabei aus PTFE, welches gegenüber Silikon gute Gleiteigenschaften hat, so daß einerseits das Einführen der Isolierung 9 in die mit dem röhrchenförmigen Teil des Einführelementes 8 ausgekleidete Innenhöhlung 4 der Isolierhülse 6 und andererseits anschließend das Herausziehen des Einführelementes 8 entsprechend leichtgängig sind.

An den Auszugenden 13 des Einführelementes 8 sind im Ausführungsbeispiel Greifhilfen in Form von gegenüber diesen Enden 13 überstehenden Verdickungen 16, Vorsprüngen od.dgl. Greifteilen vorgesehen. Somit wird für das Entfernen des Einführelementes 8 kein Werkzeug benötigt.

Zwischen dem von dem Herzschrittmacher abgewandten Ende 3b des Steckerstiftes 3 und der zur Aufnahme der Isolierung 9 des Elektrodenzuleitungsendes 5 dienenden Fortsetzung 10 der Isolierhülle 6 ist im Ausführungsbeispiel ein Zwischenabschnitt 17 der Isolierhülle 6 vorgesehen, der auch die Innenhöhlung 4 aufweist, die dabei aber in diesem Abschnitt 17 einen Querschnitt hat, der dem Außenquerschnitt der abisolierten Elektroden 2 oder Elektrodenwendel etwa entspricht, also kleiner als der Querschnitt der Innenhöhlung 4 im Bereich der Fortsetzung 10 ist. In Fig. 2 wird deutlich, daß in diesem Zwischenabschnitt 17 auch ein abisolierter Bereich des Endes 5 der Elektrodenzuleitung 2 anzuordnen ist und daß die Abstufung 18 am Übergang von dem größeren Innenquerschnitt der Innenhöhlung 4 der Isolierhülle 6 zu dem nächst kleineren als Anschlag für die radial gegenüber der Elektrode 2 überstehende Elektrodenisolierung 9 dient. Es leuchtet ein, daß somit der unmittelbar an den Steckerstift 3 anschließende Teil der Steckvorrichtung 1 in Gebrauchsstellung flexibel ist, so daß die vor allem beim Gebrauch auftretenden Relativbewegungen zwischen Elektrodenzuleitung 2 und Herzschrittmacher problemlos möglich bleiben.

Das Klemmwerkzeug 11 hat ein mit der Stecköffnung 12 versehenes Haltestück 19, dessen Stecköffnung 12 auf das Einsteckende 3a des Steckerstiftes 3 paßt und vor dem Einstekken des Steckerstiftes 3 in den Herzschrittmacher von diesem abziehbar ist. Der Innenquerschnitt dieser Stecköffnung 12 des Haltestückes 19 umschließt die Außenseite des Steckerstiftes 3 in einem Schiebe- oder Klemmsitz, so daß sich gemäß Fig. 5 zwischen der Wandung dieser Stecköffnung 12 und der Außenseite des Steckerstiftes 3 praktisch kein Zwischenraum ergibt. An dem Haltestück 19 ist nun wenigstens ein radial bis in den Querschnitt der Stecköffnung 12 verstellbares Klemmelement 20 vorgesehen, welches mit Hilfe von Gewindegängen 20a relativ zu dem Haltestück 19 verstellbar ist und an seinem außerhalb des Haltestückes 19 befindlichen Ende einen Drehgriff 21 hat. Im Ausführungsbeispiel sind dabei zwei einander an dem Haltestück 19 gegenüberliegende und miteinander fluchtende Klemmelemente 20 der vorbeschriebenen Art vorgesehen, so daß eine symmetrische Kraftaufbringung beim Verklemmen und dabei auch die Anbringung zweier Klemmstellen möglich ist.

Die Klemmelemente 20 sind an ihrem in den Querschnitt der Stecköffnung 12 des Haltestückes 19 verstellbaren Ende angespitzt, wobei ihre Spitze 22 Kegelform hat, so daß die Klemmelemente 20 auch beim Eindringen in die Stecköffnung 12 und beim radialen Verformen des Steckerstiftes 3 drehbar bleiben, so daß die Verformung genügend weit fortgesetzt werden kann und nicht durch sich selbst gebremst wird. Die Drehgriffe 21 erlauben dabei diese Verformung ohne zusatzliches Werkzeug.

Damit die Verformung nicht zu weit geht und der Steckerstift 3 und/oder das abisolierte Zuleitungsende 5 durch die Betätigung der Klemmelemente bzw. Klemmschrauben 20 beschädigt werden kann, ist zwischen der Außenseite des Haltestückes 19 und einem Anschlag, im Ausführungsbeispiel dem Drehgriff 21 des Klemmelementes 20 ein die radiale Verstellung in das Haltestück 19 und dessen Stecköffnung 12 hinein begrenzender Abstandhalter 23 zweckmäßig und gemäß 5 und 6 auch vorgesehen. Der Benutzer kann dann sicher sein, daß nach dem Einschrauben der Klemmelemente 20 bis zum Anschlag gegen diesen Abstandhalter 23 die "richtige" Verklemmung zwischen Steckerstift 3 und Elektrodenzuleitungsende 5 hergestellt ist.

Die Steckvorrichtung 1 kann dabei in vorteilhafter Weise mit dem vorstehend beschriebenen Klemmwerkzeug 11 vorgefertigt lösbar verbunden sein, wobei dies in vorteilhafter Weise mit Hilfe der Klemmelemente 20 geschehen kann, die dabei den Steckerstift 3 reibschlüssig, aber unter seiner Verformungsschwelle beaufschlagen. Somit kann die gesamte Steckvorrichtung 1 zusammen mit diesem form- und kraftschlüssig an ihr angeordneten Klemmwerkzeug 11 gelagert und geliefert und in dieser Weise vorbereitet der Benutzung zugeführt werden.Dadurch wird auch die eigentliche Operation beim Befestigen der Steckvorrichtung 1 an einer schon implantierten Elektrode 2 verkürzt.

Dabei ist im Ausführungsbeispiel an dem vorzugsweise als Haltezylinder ausgebildeten Haltestück 19 ein vorzugsweise durchsichtiges Schutzröhrchen 24 angeschlossen, welches wenigstens einen Teil der Isolierhülle 6 umschließt und im Ausführungsbeispiel bis über das dem Steckerstift 3 abgewandte Ende dieser Isolierhülle 6 reicht, sie also beim Transport und der Lagerung gegen Beschädigungen abschirmt und schützt und beim Manipulieren der Steckvorrichtung 1 vor dem Entfernen des Klemmwerkzeuges 11 dessen Handhabung erleichtert.

Die gesamte Steckvorrichtung 1 läßt sich gemäß den Figuren 1 bis 6 sehr schnell und einfach an einer Elektrodenzuleitung 2 befestigen, die evtl. schon implantiert sein kann:
Zunächst wird ein Stilett 25 von der späteren Einsteckseite her durch die Innenöffnung 7 des Steckerstiftes 3 eingeschoben. Auf dieses Stilett kann dann die wendelförmige, in ihrem Inneren ebenfalls hohle Elektrodenzuleitung 2 gemäß dem Pfeil Pf 2 in Fig. 1 aufgeschoben werden bzw. kann natürlich auch umgekehrt die ganze Steckvorrichtung 1 in entgegengesetzter Richtung über dieses Elektrodenzuleitungsende 5 geschoben werden. Dabei sorgt das Stilett 25 für eine innere Führung, die somit auch das abisolierte Elektrodenzuleitungsende 5 sicher bis in und durch den Steckerstift 3 führt.

Dabei sorgt das Einführelement 8 dafür, daß auch der nicht mehr abisolierte Bereich 5a der Elektrode 2 in die Isolierhülle 6 gelangen kann, wie es Fig. 2 zeigt.

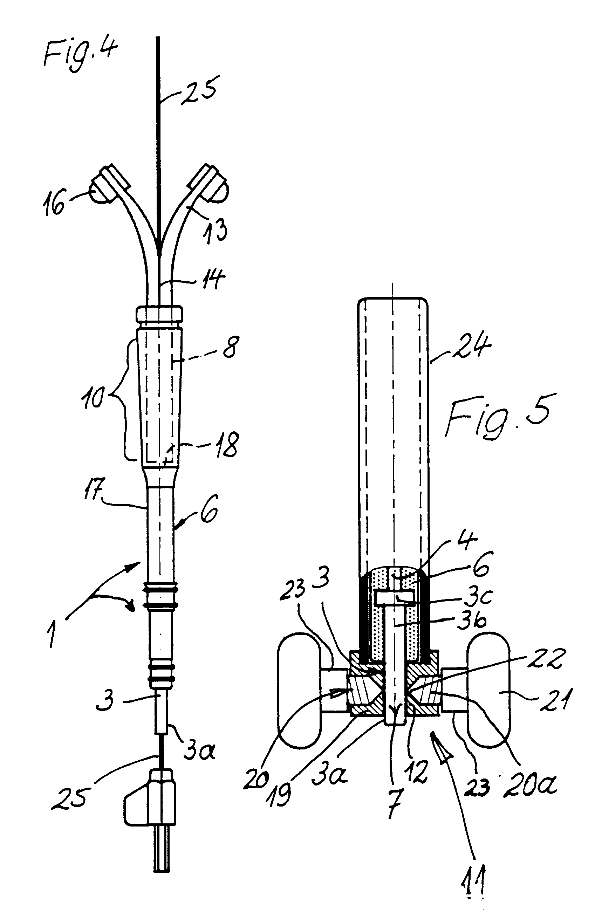

Fig. 4 deutet dabei die Lage des Stilettes 25 vor dem Verbinden der Steckvorrichtung 1 mit dem Elektrodenzuleitungsende 5 an, wobei in Fig. 4 das Klemmwerkzeug 11 weggelassen ist.

Ist das Elektrodenzuleitungsende 5 in seine Gebrauchslage eingeschoben, wobei die Isolierung 9 an der Abstufung 18 der Innenhöhlung 4 der Isolierhülle 6 zu liegen kommt, können nun gemäß Fig. 2 die Drehgriffe 21 der Klemmelemente 20 gemäß den Pfeilen Pf4 betätigt und dadurch die Klemmelemente 20 gemäß den Pfeilen Pf 1 radial nach innen verstellt werden, wodurch sich die Verklemmung zwischen Steckerstift 3 und Elektrodenzuleitungsende 5 im abisolierten Bereich und damit auch ein Formschluß zwischen Elektrodenzuleitung 2 und Steckvorrichtung 1 ergibt.

Nun kann gegebenenfalls bei noch an der Steckvorrichtung 1 angreifendem Klemmwerkzeug 11 das Einführelement 8 entsprechend den Pfeilen Pf3 in Fig. 6 herausgezogen werden, wobei das Klemmwerkzeug 11 dem Operateur eine gute Gegenhalterung entgegen den Zugkräften beim Herausziehen dieses Einführelementes 8 erlaubt, da es ja mit seinen Klemmelementen 21 praktisch formschlüssig an dem Steckerstift 3 angreift. Bei diesem Herausziehen des Einführelementes 8 wird es gleichzeitig entlang den Sollreißstellen 14 aufgetrennt, so daß es nicht nur herausgezogen, sondern gleichzeitig von der gesamten Anorndung entfernt wird. Dabei sei erwähnt, daß zuvor zumindest die Fortsetzung 10 der Isolierhülle 6 etwas aufgeweitet sein kann, um das Einstecken und auch das Entfernen des Einführelementes 8 zu erleichtern. Schrumpft dann dieser z.B. mittels chemischer Mittel aufgeweitete Bereich der Isolierhülle 6 wieder auf sein Ausgangsmaß, legt er sich fest auf die Isolierung 9 und verstärkt dadurch die Verbindung zwischen Steckervorrichtung 1 und Elektrodenzuleitungsende 5 bzw. 5a.

Dabei ist das über die Isolierhülle 6 vorstehende Einsteckende 3a des Steckerstiftes 3 zum Verklemmen mit dem Elektrodenzuleitungsende radial verformbar, so daß es nach einer solchen radialen Verformung formschlüssig, kraftschlüssig und auch elektrisch leitend mit dem Elektrodenzuleitungsende 5 verbunden ist.

Zuletzt kann dann das Stilett herausgezogen und ein evtl. über das Einsteckende 3a des Steckerstiftes 3 überstehender Rest des Elektrodenzuleitungsendes 5 abgeschnitten werden. Die Anbringung der erfindungsgemäßen Steckervorrichtung 1 kann also sehr schnell und einfach durchgeführt werden, wobei sich der große Vorteil ergibt, daß der Steckerstift 3 und die gesamte Steckvorrichtung 1 eine relativ geringe radiale Ausdehnung hat und dennoch eine feste Verbindung mit dem Elektrodenzuleitungsende 5 mit Hilfe der Kombination der besonderen Ausgestaltung der eigentlichen Steckvorrichtung 1 und auch des Klemmwerkzeuges 11 sowie des Einführelementes 8 gestattet. Es ist auf diese Weise möglich, Steckerstifte mit einem Außendurchmesser von etwa 3 mm an Elektrodenzuleitungsenden 5 anzuschließen, so daß die Steckvorrichtung 1 in moderne Herzschrittmacher mit entsprechend eng bemessenen Einstecköffnungen paßt.

Die Steckvorrichtung 1 für eine Herzschrittmacher-Elektrodenzuleitung 2 hat einen in Längsrichtung hohlen Steckerstift 3 für den Anschluß an einen Herzschrittmacher und eine in Verlängerung des Steckerstiftes 3 eine Einstecköffnung 4 zur Aufnahme des freien Endes 5 der Elektrodenzuleitung 2 aufweisende Isolierhülle 6, wobei die elektrisch leitende Verbindung durch Verklemmen hergestellt wird. Dabei paßt die Innenlängshöhlung 7 des Steckerstiftes 3 über das abisolierte Elektrodenzuleitungsende 5 und ist zum Verklemmen damit radial verformbar. An seinem dem Einsteckende 3a abgewandten Ende 3b trägt der Steckerstift 3 die Isolierhülle 6 mit einem Einführelement 8 für den isolierten Bereich 5a des Elektrodenzuleitungsendes 5, welche die Innenhöhlung 4 der Isolierhülle 6 soweit auskleidet, daß die verbleibende Isolierung 9 des Bereiches 5a leichtgängig in die Isolierhülle 6 eingeschoben werden kann. Das Einführelement 8 steht über das Ende der Isolierhülle 6 vor und kann nach der Montage leicht herausgezogen werden. In radialer Richtung einen Platzbedarf aufweisende Klemmschrauben werden in vorteilhafter Weise vermieden, so daß die radialer Ausdehnung der Steckervorrichtung 1 entsprechend gering ausfällt.

## Patentansprüche

1. Herzschrittmacher-Elektrodenzuleitung (2) mit einer Steckvorrichtung (1), die einen in Längsrichtung hohlen Steckerstift (3) für den Anschluß an einen Herzschrittmacher und eine in Verlängerung des Steckerstiftes (3) eine Einstecköffnung (4) zur Aufnahme des freien Endes (5) der Elektrodenzuleitung (2) aufweisende Isolierhülle (6) hat, wobei das Elektrodenzuleitungsende (5) durch Verklemmen mit dem Steckerstift (3) elektrisch leitend verbindbar ist, **dadurch gekennzeichnet**, daß das freie, abisolierte Elektrodenzuleitungsende (5) in die Innenlängshöhlung (7) des Steckerstiftes (3) einsteckbar und zum Verklemmen damit wenigstens bereichsweise radial verformbar ist, daß der Steckerstift (3) an seinem dem Einsteckende (3a) abgewandten Ende (3b) eine Isolierhülle (6) mit einem Einführelement (8) für den isolierten Bereich (5a) des Elektrodenzuleitungsendes (5) trägt, daß in eine Fortsetzung (10) der Isolierhülle (6) das deren Innenhöhlung (4) wenigstens teilweise auskleidende Einführelement (8) aus einem Werkstoff eingesetzt ist, gegenüber welchem die Außenisolierung (9) der Elektrodenzuleitung (2) gleitfähig ist, daß das Einführelement (8) über das dem Einsteckende (3a) abgewandte rückwärtige Ende der Isolierhülle (6) vorsteht und nach dem Einschieben des Elektrodenzuleitungsendes (5) relativ zu diesem und zu der Isolierhülle (6) entgegen der Einsteckrichtung herausziehbar ist.

2. Herzschrittmacher-Elektrodenzuleitung nach Anspruch 1, dadurch gekennzeichnet, daß die Innenlängshöhlung (7) des Steckerstiftes (3) einen über den Außenquerschnitt des abisolierten Elektrodenzuleitungsendes (5) steck- oder schiebbaren Querschnitt hat, daß der von dem Einsteckende (3a) abgewandte rückwärtige Abschnitt (3b) des Steckerstiftes (3) von der Isolierhülle (6) umschlossen ist, daß sich die Isolierhülle (6) über das dem Einsteckende (3a) abgewandte Ende (3b) des Steckerstiftes (3) fortsetzt und im Inneren dieser Fortsetzung (10) eine Innenhöhlung (4) hat, deren Querschnitt dem Außenquerschnitt der mit Isolierung (9) versehenen Elektrodenzuleitung (2) etwa entspricht.

3. Herzschrittmacher-Elektrodenzuleitung nach Anspruch 1 oder 2, dadurch gekennzeichnet, daß zum Verformen des Steckerstiftes (3) für seine Verklemmung mit dem in ihm steckenden abisolierten Elektrodenzuleitungsende ein Klemmwerkzeug (11) mit einer Öffnung (12) vorgesehen ist, in welche der Steckerstift (3) und sein zu verformender Bereich einführbar ist.

4. Herzschrittmacher-Elektrodenzuleitung nach einem der Ansprüche 1 bis 3, dadurch gekennzeichnet, daß das Einführelement (8) aus zwei trennbaren oder einzelnen Teilen (8a, 8b) besteht, deren jeder ein aus der Isolierhülle (6) vorstehendes Auszugende (13) hat und in Gebrauchsstellung die Isolierung (9) des Elektrodenzuleitungsendes (5a) von der Innenseite der Isolierhülle (6) abschirmt.

5. Herzschrittmacher-Elektrodenzuleitung nach einem der Ansprüche 1 bis 3, dadurch gekennzeichnet, daß das Einführelement (8) aus einem einstückigen röhrchenförmigen Teil mit wenigstens einer, vorzugsweise zwei in dessen Längsrichtung verlaufender Sollreißstelle (14) gebildet ist, welche durch das Herausziehen gleichzeitig auftrennbar ist.

6. Herzschrittmacher-Elektrodenzuleitung nach Anspruch 5, dadurch gekennzeichnet, daß wenigstens der röhrchenförmige Teil des Einführelementes (8) aus PTFE besteht.

7. Herzschrittmacher-Elektrodenzuleitung nach Anspruch 4, dadurch gekennzeichnet, daß an den Auszugenden (13) des Einführelementes (8) Greifhilfen, insbesondere gegenüber diesen Enden (13) überstehende Verdickungen (16), Vorsprünge oder dergleichen vorgesehen sind.

8. Herzschrittmacher-Elektrodenzuleitung nach einem der Ansprüche 1 bis 7, dadurch gekennzeichnet, daß zwischen dem von dem Herzschrittmacher abgewandten Ende (3b) des Steckerstiftes (3) und der zur Aufnahme der Isolierung (9) des Elektrodenzuleitungsendes (5) dienenden Fortsetzung (10) der Isolierhülle (6) ein Zwischenabschnitt (17) der Isolierhülle (6) vorgesehen ist, dessen Innenhöhlung (4) einen Querschnitt hat, der dem Außenquerschnitt der abisolierten Elektrode (2) oder Elektrodenwendel etwa entspricht.

9. Herzschrittmacher-Elektrodenzuleitung nach einem der Ansprüche 1 bis 8, dadurch gekennzeichnet, daß ein mit einer Stecköffnung (12) versehenes Haltestück (19) vorgesehen ist, dessen Stecköffnung (12) auf das Einsteckende (3a) des Steckerstiftes (3) paßt und vor dem Einstecken des Steckerstiftes (3) in den Herzschrittmacher von dem Steckerstiftende abziehbar ist, daß der Innenquerschnitt der Stecköffnung (12) des Haltestückes (19) die Außenseite des Steckerstiftes (3) in einem Schiebe- oder Klemmsitz umschließt und daß an dem Haltestück (19) wenigstens ein radial bis in den Querschnitt der Stecköffnung (12) verstellbares Klemmelement (20) vorgesehen ist.

10. Herzschrittmacher-Elektrodenzuleitung nach Anspruch 9, dadurch gekennzeichnet, daß das radial verstellbare Klemmelement (20) mit Hilfe von Gewindegängen (20a) relativ zu dem Haltestück (19) verstellbar ist und vorzugsweise an einem außerhalb des Haltestückes (19) befindlichen Bereich oder Ende einen Drehgriff (21) hat.

11. Herzschrittmacher-Elektrodenzuleitung nach Anspruch 9 oder 10, dadurch gekennzeichnet, daß das/die Klemmelemente (20) am in den Querschnitt der Stecköffnung (12) des Haltestückes (19) verstellbaren Ende angespitzt ist/sind und daß das Klemmelement (20) auch beim Eindringen in die Stecköffnung (12) und radialen Verformen des Steckerstiftes (3) drehbar ist.

12. Herzschrittmacher-Elektrodenzuleitung nach einem der Ansprüche 9 bis 11, dadurch gekennzeichnet, daß zwei einander an dem Haltestück (19) gegenüberliegende und vorzugsweise etwa miteinander fluchtende Klemmelemente (20) vorgesehen sind.

13. Herzschrittmacher-Elektrodenzuleitung nach einem der Ansprüche 10 bis 12, dadurch gekennzeichnet, daß zwischen der Außenseite des Haltestückes (19) und einem Anschlag, insbesondere dem Drehgriff (21) des Klemmelementes (20), ein die radiale Verstellung des Klemmelementes in das Haltestück (19) und dessen Stecköffnung (12) hinein begrenzender Abstandhalter (23) vorgesehen ist.

14. Herzschrittmacher-Elektrodenzuleitung nach einem der Ansprüche 9 bis 13, dadurch gekennzeichnet, daß das Haltestück (19) mit dem Steckerstift und dessen Isolierhülle vorgefertigt lösbar insbesondere mit Hilfe des/der Klemmelemente verbunden ist und daß das/die Klemmelemente dabei den Steckerstift reibschlüssig, unterhalb seiner Verformungsschwelle beaufschlagen.

15. Herzschrittmacher-Elektrodenzuleitung nach einem der Ansprüche 9 bis 14, dadurch gekennzeichnet, daß an dem Haltestück (19), insbesondere einem Haltezylinder ein vorzugsweise durchsichtiges Schutzröhrchen (24) angeschlossen ist, welches wenigstens einen Teil der Isolierhülle (6) umschließt und vorzugsweise bis über das Ende der Isolierhülle (6) reicht.

16. Herzschrittmacher-Elektrodenzuleitung nach einem der Ansprüche 1 bis 15, dadurch gekennzeichnet, daß eine Abstufung (18) am Übergang von dem größeren Innenquerschnitt der Isolierhülle (6) zu dem nächst kleineren als Anschlag für die radial gegenüber der Elektrode (2) überstehende Elektrodenisolierung (9) dient.

17. Herzschrittmacher-Elektrodenzuleitung nach einem der vorstehenden Ansprüche, dadurch gekennzeichnet, daß das über die Isolierhülle (6) vorstehende Einsteckende (3a) des Steckerstiftes (3) zum Verklemmen und elektrischen Verbinden mit dem in seine Innenlängshöhlung (7) eingeführten Elektrodenzuleitungsende (5) radial verformbar ist.

## Claims

1. A pacemaker electrode lead (2) including a coupler (1) having a plug pin (3) which is hollow in the longitudinal direction and serves to establish the connection to a cardiac pacemaker, and an insulating cover (6) having in extension of said plug pin (2) an inlet opening (4) to receive the free end (5) of the electrode lead (2), wherein the end (5) of the electrode lead is electroconductively connectable to the plug pin (3) by clamping, **characterized in that** the free, bared end (5) of the electrode lead is insertable into the inner longitudinal cavity (7) of the plug pin (3) and for clamping thereto is at least locally radially deformable, that the plug pin (3) carries at its end (3b) remote from the plug-in end (3a) an insulating cover (6) with a guide (8) for introducing the insulated area (5a) of the end (5) of the electrode lead, that the guide (8) is fitted into a continuation (10) of the insulating cover (6) and at least partly lines the inner cavity (4) thereof and is made of a material relative to which the outer insulation (9) of the electrode lead (2) is slidable, that the guide (8) projects beyond the distal end remote from the plug-in end (3a) of the insulating cover (6) and, after the end (5) of the electrode lead has been slipped in, is withdrawable relative thereto and to the insulating cover (6) in a direction counter to that of insertion.

2. A pacemaker electrode lead as claimed in claim 1, characterized in that the inner longitudinal cavity (7) of the plug pin (3) has a cross section capable of slipping or sliding over the outer cross section of the bared end (5) of the electrode lead, that the distal plug pin portion (3b) remote from the plug-in end (3a) is surrounded by the insulating cover (6), that the insulating cover (6) continues beyond the plug pin end (3b) remote from the plug-in end (3a) and has inside said continued portion (10) an inner cavity (4) the cross section of which corresponds approximately to the outer cross section of the electrode lead (2) provided with insulation (9).

3. A pacemaker electrode lead as claimed in claim 1 or claim 2, characterized in that to deform the plug pin (3) for clamping it to the bared end of the electrode lead placed therein, a clamping tool (11) is provided having an opening (12) into which the plug pin and area thereof to be deformed is insertable.

4. A pacemaker electrode lead as claimed in any one of claims 1 to 3, characterized in that the guide (8) consists of two separable or discrete sections (8a, 8b), each of which has a pull-out end (13) projecting from the insulating cover (6) and in the position of use shields the insulation (9) of the end (5a) of the electrode lead from the inside of the insulating cover (6).

5. A pacemaker electrode lead as claimed in any one of claims 1 to 3, characterized in that the guide (8) is formed by an integral, tubular part having extending in its longitudinal direction at least one, preferably two predetermined tearing points (14) simultaneously severable by extracting the guide.

6. A pacemaker electrode lead as claimed in claim 5, characterized in that at least the tubular part of the guide (8) consists of PTFE.

7. A pacemaker electrode lead as claimed in claim 4, characterized in that the pull-out ends (13) of the guide (8) are provided with grips, particularly protuberances (16), projections or the like projecting from said ends (13).

8. A pacemaker electrode as claimed in any one of claims 1 to 7, characterized in that the insulating cover (6) is provided with an intermediate portion (17) between the plug pin end (3b) remote from the cardiac pacemaker and the portion (10) continuing the insulating cover (6) and serving to receive the insulation (9) of the end (5) of the electrode lead, the inside cavity (4) of said intermediate portion (17) having a cross section corresponding approximately to the outer cross section of the bared electrode (2) or electrode coil.

9. A pacemaker electrode lead as claimed in any one of claims 1 to 8, characterized in that provision is made for a holder (19) provided with an opening (12) which fits on the plug-in end (3a) of the plug pin (3) and is withdrawable from said end before the plug pin (3) is introduced into the pacemaker, that the inside cross section of the holder opening (12) surrounds the exterior of the plug pin (3) in a sliding or press fit and that the holder (19) is provided with at least one clamping element (20) adjustable radially right into the cross section of the holder opening (12).

10. A pacemaker electrode lead as claimed in claim 9, characterized in that the radially adjustable clamping element (20) is adjustable relative to the holder (19) with the aid of threads (20a) and has a twist grip (21) preferably at a zone or end situated outside the holder (19).

11. A pacemaker electrode lead as claim in claim 9 or claim 10, characterized in that the clamping element(s) (20) is/are pointed at the end adjustable into the cross section of the opening (12) of the holder (19) and that the clamping element (20) is rotatable even as it penetrates the opening (12) and as the plug pin (3) is radially deformed.

12. A pacemaker electrode lead as claimed in any one of claims 9 to 11, characterized in that provision is made for two clamping elements (20) which are mutually opposed at the holder (19) and are preferably approximately in alignment.

13. A pacemaker electrode lead as claimed in any one of claims 10 to 12, characterized in that a spacer (23) is provided between the exterior of the holder (19) and a stop, particularly the twist grip (21) of the clamping element (20), and limits the radial adjustment of the clamping element into the holder (19) and holder opening (12).

14. A pacemaker electrode lead as claimed in any one of claims 9 to 13, characterized in that the holder (19) is preassembled so as to be detachably connected to the plug pin and insulating cover thereof particularly with the aid of the clamping element(s), and that the clamping element(s) engage the plug pin frictionally, under its threshold of deformation.

15. A pacemaker electrode lead as claimed in any one of claims 9 to 14, characterized in that the holder (19), particularly a cylindrical holding member, has connected thereto a preferably transparent, protective tubule (24) which surrounds at least a portion of the insulating cover (6) and preferably extends over the end of the insulating cover (6).

16. A pacemaker electrode lead as claimed in any one of claims 1 to 15, characterized in that a graduation (18) at the transition from the larger inner cross section of the insulating cover (6) to the next smaller one serves as a stop for the electrode insulation (9) projecting radially relative to the electrode (2).

17. A pacemaker electrode lead as claimed in any one of the preceding claims, characterized in that the plug-in end (3a) of the plug pin (3) projecting beyond the insulating cover (6) is radially deformable for being clamped and electrically connected to the end (5) of the electrode lead inserted into the inner longitudinal cavity (7) of said plug pin (3).

## Revendications

1. Conducteur d'électrode (2) de stimulateur cardiaque, avec un dispositif de connexion (1) qui possède une broche de connexion (3) pour le raccordement à un stimulateur cardiaque et une gaine isolante (6) présentant, dans le prolongement de la broche de connexion (3), une ouverture d'enfichage (4) pour recevoir l'extrémité libre (5) du conducteur d'électrode (2), l'extrémité (5) du conducteur d'électrode pouvant être reliée en conduction électrique par serrage à la broche de connexion (3), **caractérisé** en ce que l'extrémité libre dénudée (5) du conducteur d'électrode peut être enfichée dans la cavité longitudinale intérieure (7) de la broche de connexion (3) et être ainsi déformée radialement au moins sectoriellement pour la liaison par serrage, en ce que la broche de connexion (3) porte, à son extrémité (3b) opposée à l'extrémité de connexion (3a), une gaine isolante (6) avec un élément d'introduction (8) pour la région isolée (5a) de l'extrémité (5) du conducteur d'électrode, en ce que l'élément d'introduction (8) est inséré dans un prolongement (10) de la gaine isolante (6) dont il garnit au moins partiellement la cavité intérieure (4) et est réalisé en un matériau contre lequel peut glisser l'isolation extérieure (9) du conducteur d'électrode (2), et en ce que l'élément d'introduction (8) dépasse de l'extrémité arrière, opposée à l'extrémité de connexion (3a), de la gaine isolante (6) et, à la suite de l'enfichage de l'extrémité (5) du conducteur d'électrode, peut être retiré en le tirant, par rapport à cette dernière et à la gaine isolante (6), à l'encontre de la direction d'enfichage.

2. Conducteur d'électrode de stimulateur cardiaque selon la revendication 1, **caractérisé** en ce que la cavité longitudinale intérieure (7) de la broche de connexion (3) possède une section permettant l'emboîtement ou l'enfilage sur la section extérieure de l'extrémité dénudée (5) du conducteur d'électrode, en ce que la partie arrière (3b) de la broche de connexion (3), opposée à l'extrémité de connexion, est entourée par la gaine isolante (6), et en ce que la gaine isolante (6) se prolonge au delà de l'extrémité (3b) de la broche de connexion (3) opposée à l'extrémité de connexion (3a) et possède, à l'intérieur de ce prolongement (10), une cavité intérieure (4) dont la section correspond approximativement à la section extérieure du conducteur d'électrode (2) pourvu d'une isolation (9).

3. Conducteur d'électrode de stimulateur cardiaque selon la revendication 1 ou 2, **caractérisé** en ce qu'afin de déformer la broche de connexion (3) pour sa liaison par serrage avec l'extrémité dénudée du conducteur d'électrode enfichée dans cette broche, il est prévu un outil de serrage (11) avec une ouverture (12) dans laquelle peut être introduite la broche de connexion (3) et sa région à déformer.

4. Conducteur d'électrode de stimulateur cardiaque selon l'une des revendications 1 à 3, **caractérisé** en ce que l'élément d'introduction (8) comprend deux parties séparables ou séparées (8a, 8b), dont chacune possède une extrémité d'extraction (13) dépassant de la gaine isolante (6) et, en position d'utilisation, protège l'isolation (9) de l'extrémité (5a) du conducteur d'électrode vis-à-vis du côté intérieur de la gaine isolante (6).

5. Conducteur d'électrode de stimulateur cardiaque selon l'une des revendications 1 à 3, **caractérisé** en ce que l'élément d'introduction (8) comprend une partie tubulaire d'un seul tenant avec au moins un et de préférence deux points de rupture privilégiée (14) s'étendant dans sa direction longitudinale, qui peuvent être simultanément sectionnés lors de l'extraction.

6. Conducteur d'électrode de stimulateur cardiaque selon la revendication 5, **caractérisé** en ce qu'au moins la partie tubulaire de l'élément d'introduction (8) est réalisée en polytétrafluoréthylène (PTFE).

7. Conducteur d'électrode de stimulateur cardiaque selon la revendication 4, **caractérisé** en ce que des aides à la préhension sont prévues sur les extrémités d'extraction (13) de l'élément d'introduction (8), notamment des épaississements (16), saillies ou similaires faisant saillie sur ces extrémités (13).

8. Conducteur d'électrode de stimulateur cardiaque selon l'une des revendications 1 à 7, **caractérisé** en ce qu'est prévu, entre l'extrémité (3b) de la broche de connexion (3) opposée au stimulateur cardiaque et le prolongement (10) de la gaine isolante (6) servant à recevoir l'isolation (9) de l'extrémité (5) du conducteur d'électrode, un tronçon intermédiaire (17) de la gaine isolante (6) dont la cavité intérieure (4) possède une section qui correspond approximativement à la section extérieure du conducteur d'électrode dénudé (2) ou encore du filament d'électrode.

9. Conducteur d'électrode de stimulateur cardiaque selon l'une des revendications 1 à 8, **caractérisé** en ce qu'est prévu un organe de retenue (19), qui est pourvu d'une ouverture d'emboîtement (12) s'adaptant sur l'extrémité de connexion (3a) de la broche de connexion (3) et qui peut être retiré de l'extrémité de la broche de connexion avant l'enfichage de la broche de connexion (3) dans le stimulateur cardiaque, en ce que la section intérieure de l'ouverture d'emboîtement (12) de l'organe de retenue (19) entoure le côté extérieur de la broche de connexion (3) en ajustement glissant ou serré, et en ce qu'est prévu, sur l'organe de retenue (19), au moins un élément de serrage (20) pouvant être déplacé radialement jusque dans la section de l'ouverture d'emboîtement (12).

10. Conducteur d'électrode de stimulateur cardiaque selon la revendication 9, **caractérisé** en ce que l'élément de serrage (20) pouvant être déplacé radialement peut être déplacé par rapport à l'organe de retenue (19) à l'aide de filets (20a) et possède, de préférence sur une région ou à une extrémité située en dehors de l'organe de retenue (19), une poignée tournante (21).

11. Conducteur d'électrode de stimulateur cardiaque selon la revendication 9 ou 10, **caractérisé** en ce que le ou les éléments de serrage (20) sont taillés en pointe à l'extrémité pouvant être déplacée dans la section de l'ouverture d'emboîtement (12) de l'organe de retenue (19), et en ce que l'élément de serrage (20) est également rotatif lors de l'enfoncement dans l'ouverture d'emboîtement (12) et de la déformation radiale de la broche de connexion (3).

12. Conducteur d'électrode de stimulateur cardiaque selon l'une des revendications 9 à 11, **caractérisé** en ce que sont prévus deux éléments de serrage (20) se faisant face sur l'organe de retenue (19) et de préférence approximativement en alignement mutuel.

13. Conducteur d'électrode de stimulateur cardiaque selon l'une des revendications 10 à 12, **caractérisé** en ce qu'est prévu, entre le côté extérieur de l'organe de retenue (19) et une butée, notamment la poignée tournante (21) de élément de serrage (20), un écarteur (23) limitant le déplacement radial de l'élément de serrage à l'intérieur de l'organe de retenue (19) et de son ouverture d'emboîtement (12).

14. Conducteur d'électrode de stimulateur cardiaque selon l'une des revendications 9 à 13, **caractérisé** en ce que l'organe de retenue (19) est préassemblé de façon démontable à la broche de connexion (3) et à sa gaine isolante, notamment à l'aide du ou des éléments de serrage, et en ce que le ou les éléments de serrage sollicitent alors la broche de connexion par pression en dessous de son seuil de déformation.

15. Conducteur d'électrode de stimulateur cardiaque selon l'une des revendications 9 à 14, **caractérisé** en ce qu'est raccordé à l'organe de retenue (19), qui est notamment un cylindre de retenue, un tube de protection (24), de préférence transparent, qui entoure au moins une partie de la gaine isolante (6) et s'étend de préférence jusqu'au delà de l'extrémité de la gaine isolante (6).

16. Conducteur d'électrode de stimulateur cardiaque selon l'une des revendications 1 à 15, **caractérisé** en ce qu'un gradin (18), à la transition entre la plus grande section intérieure de la gaine isolante (6) et la section plus petite consécutive, sert de butée pour l'isolation d'électrode (9) dépassant radialement par rapport au conducteur d'électrode (2).

17. Conducteur d'électrode de stimulateur cardiaque selon l'une des revendications précédentes, **caractérisé** en ce que l'extrémité de connexion (3a) de la broche de connexion (3), qui dépasse de la gaine isolante (6), peut être déformée radialement en vue de sa liaison, électrique et avec serrage, avec l'extrémité (5) du conducteur d'électrode qui est introduite dans la cavité longitudinale intérieure (7) de la broche.
